# EUROPEAN PATENT APPLICATION

(11) **EP 4 588 421 A1**
(43) Date of publication of application: **23.07.2025**
(21) Application number: 25152943.4
(22) Date of filing: 20.01.2025
(51) Int. Cl.: A61B 1/00, A61B 1/005

(54) **FLEXIBLE SHAFT, SURGICAL INSTRUMENT, METHOD FOR MANUFACTURING A FLEXIBLE SHAFT AND METHOD FOR ASSEMBLING A STEERING WIRE TO A FLEXIBLE SHAFT**

(30) Priority: 22.01.2024 DE 102024101692
(71) Applicant: Karl Storz SE & Co. KG, 78532 Tuttlingen (DE)
(72) Inventor: Juhanson, Timo, 78532 Tuttlingen (DE); Poldmann, Mauno, 78532 Tuttlingen (DE)

(57) **Abstract**

The present invention provides a flexible shaft (1) for a surgical instrument, in particular for a flexible endoscope, comprising: a distal end (2) and a proximal end (3); and a plurality of shaft sections (4), which are arranged in series between the distal end (2) and the proximal end (3); wherein the plurality of shaft sections (4) is articulatable at least in one plain and comprises a through-opening (5), which is configured for receiving a working channel and an optical image device, wherein the through-opening (5) is further configured for receiving at least two steering wires (6), wherein the through-opening (5) extends through the plurality of shaft sections (4) from the proximal end (3) to the distal end (2). Further the present invention provides a surgical instrument comprising such a flexible shaft as well as a method for manufacturing a flexible shaft for a surgical instrument and a method for assembling a steering wire to a flexible shaft for a surgical instrument.

## Description

### TECHNICAL FIELD

The present invention pertains to a flexible shaft for a surgical instrument as well as a surgical instrument with such a flexible shaft. Furthermore, the present invention pertains to a method for manufacturing a flexible shaft for a surgical instrument and a method for assembling a steering wire to a flexible shaft for a surgical instrument.

### BACKGROUND

Flexible surgical tube shaft instruments such as flexible endoscopes are used for a variety of applications in medicine and technology. Such flexible endoscopes comprise a flexible elongated shaft which is suitable for insertion into a cavity, such as an internal body cavity or a cavity of a technical object. As a rule, an endoscope lens is arranged at the tip of the endoscope shaft to generate an image of a scene in the observed cavity. To record and transmit the endoscopic image from the distal (i.e. far from the observer) to the proximal (i.e. close to the observer) end region of the endoscope, an ordered bundle of optical fibers running inside the shaft can be provided, for example, or an electronic image sensor, such as a CCD chip, which is arranged in the region of the distal end of the shaft and whose signals are transmitted to the proximal end region via electrical lines running inside the shaft. Since there is usually not enough light in the observed cavity, a light guide system can also be arranged inside the shaft to transport light to the distal end of the endoscope, where it is used to illuminate the cavity. Further, the endoscope shaft may include one or more working channels for passing endoscopic working instruments from the proximal to the distal end portion of the shaft for performing manipulations within the cavity.

Flexible surgical tube shaft instruments are also known which comprise a flexible elongated shaft which is also suitable for insertion into a cavity, such as an internal body cavity or a cavity of a technical object. Such a flexible endoscopic instrument can be used to carry out manipulations in the cavity and for this purpose can be designed, for example, as a grasping instrument for grasping and manipulating tissue or objects in the cavity inside the body or in the cavity of a technical object. For this purpose, a tool is arranged at the distal end of the flexible shaft, which can be operated from the proximal end of the shaft via a transmission means running inside the shaft. Such a flexible endoscopic instrument usually does not have its own optics for recording an endoscopic image, but can be used in particular together with a flexible endoscope.

It is often desirable to be able to angle the distal end of the shaft, i.e. the tip of the endoscope or endoscopic instrument, in order to facilitate the insertion of the endoscope or endoscopic instrument through a non-flexible endoscope through a non-rectilinear channel, to be able to move the tip within a cavity in a lateral direction and to be able to change the viewing direction of an optical system arranged in the endoscope tip or the working direction of a tool arranged at the tip of the endoscopic instrument. For this purpose, the shaft has a controllable section, in particular a controllable end section, which can be actively angled by a desired amount in a desired direction and can be controlled for this purpose from the proximal end of the endoscope or endoscopic instrument. The shaft for a flexible endoscopic instrument need not itself have a tool and a transmission means, but can for example comprise a working channel into which a flexible endoscopic working instrument which cannot be actively angled and which has such a tool can be inserted up to the distal end of the shaft and possibly beyond, so that the flexible working instrument can be angled with the aid of the shaft.

In order to enable controllable articulation of a section of a shaft of a flexible endoscope, it is known to form the latter with a basic structure comprising individual segments which can be pivoted relative to one another and which can be actuated by cable pulls or Bowden cables guided in the endoscope shaft. In particular, handwheels arranged on the endoscope handle are provided for actuation.

In document US 2005/0131279 A1 each segment of a controllable section of the endoscope shaft is pivotably mounted relative to the next or previous segment. Four cables are provided for angling the shaft section or endoscope tip in question, which are guided from a proximally arranged control device to the endoscope tip. The cables run in the edge area of the segments and are each offset by 90° to one another with respect to a longitudinal axis, so that the endoscope tip can be steered in a desired direction by rolling up the corresponding cables in the control device.

However, each cable is guided in a tiny through-hole running along the shaft such that manufacturing and in particular assembling of the flexible shaft is difficult and/or lengthy. Current one-piece injection molded shafts on the market usually have complex molds to make round cable guide holes through the whole shaft.

### DISCLOSURE OF THE INVENTION

It is an object of the present invention to provide a flexible shaft, which can be produced and assembled easily and quick.

According to the invention, this problem is solved by a flexible shaft for a surgical instrument with the features of claim 1, by a surgical instrument with the features of claim 8, a method for manufacturing a flexible shaft for a surgical instrument with the features of claim 11 and/or by a method for assembling a steering wire to a flexible shaft for a surgical instrument with the features of claim 13.

According to a first aspect of the invention, a flexible shaft for a surgical instrument, in particular for a flexible endoscope, is provided. The flexible shaft comprises a distal end and a proximal end. Further, the flexible shaft comprises a plurality of shaft sections, which are arranged in series between the distal end and the proximal end. The plurality of shaft sections is articulatable at least in one plain and comprises a through-opening, which is configured for receiving a working channel and an optical image device. The through-opening is further configured for receiving at least two steering wires, wherein the through-opening extends through the plurality of shaft sections from the proximal end to the distal end.

According to a second aspect of the invention, a flexible surgical instrument, in particular flexible endoscope, is provided. The flexible surgical instrument comprises a flexible shaft according to the first aspect of the invention arranged at a distal end of the flexible surgical instrument. Further, the flexible surgical instrument comprises an operating interface, which is arranged at the proximal end of the flexible shaft and configured to operate the flexible shaft.

According to a third aspect of the invention, a method for manufacturing a flexible shaft for a surgical instrument, in particular for manufacturing a flexible shaft according to the first aspect of the invention, is provided. The method comprises the steps of:
Primary shaping of a plurality of shaft sections, which are arranged in series between a distal end and a proximal end and comprise a through-opening, which is configured for receiving a working channel and an optical image device, wherein the through-opening is further configured for receiving at least two steering wires, wherein the through-opening extends through the plurality of shaft sections from the proximal end to the distal end.

Releasing the flexible shaft from the primary shaping tool, wherein the plurality of shaft sections is articulatable at least in one plain.

According to a fourth aspect of the invention, a method for assembling a steering wire to a flexible shaft for a surgical instrument, in particular to a flexible shaft according to the first aspect of the invention, is provided. The method comprises the steps of:
Providing a flexible shaft comprising a distal end, a proximal end, and a plurality of shaft sections, which are arranged in series between the distal end and the proximal end, wherein the plurality of shaft sections is articulatable at least in one plain and comprises a through-opening, which is configured for receiving a working channel and an optical image device, wherein the through-opening is further configured for receiving at least two steering wires, where-in the through-opening extends through the plurality of shaft sections from the proximal end to the distal end.

Guiding a first steering wire and a second steering wire through the through-opening of the plurality of shaft sections from the proximal end to the distal end.

Sliding the first steering wire in a radial direction of the flexible shaft for radially positioning the first steering wire in relation to the through-opening.

A fundamental concept of the invention is to provide a flexible vertebrae or shaft, respectively, including an inner contour or through-opening, respectively, for all main components of the flexible surgical instrument, in particular flexible endoscope. The inner contour in particular accommodates the steering wires, the working channel and the optical image device. The flexible vertebrae or shaft, respectively, corresponds to an active bending section of the flexible surgical instrument, in particular flexible endoscope. The distal end of the flexible shaft corresponds to the distal end of the flexible surgical instrument.

Furthermore, the methods disclose a way of manufacturing the vertebrae and assembling the vertebrae to the endoscope.

A particular advantage in the solution according to an aspect of the invention is that the flexible shaft comprises one inner contour or through-opening, respectively, instead of three separate openings that need to be formed in the manufacturing method. One inner contour can simplify the assembly procedure of the steering wires.

A further advantage of the present invention is that limitations of plastics to use for the flexible shaft can be reduced because the flexible shaft according to the invention does not have small features. Moreover, assembling of the small components such as the steering wires can be simplified due to the relative large inner contour compared to the typical separate openings for steering wires as known in the prior art.

The present invention can provide an easier and/or more robust construction for the vertebrae. Additionally, the present invention can provide an easier and faster assembly of the steering wires.

The operating interface and/or the surgical instrument can be made of a plastic material. For example, the surgical instrument can be completely made of a plastic material, in particular when the surgical instrument is intended to use only once.

The working channel can be inserted into a tubular shaft of the surgical instrument. The working channel is mounted or guided at least at an end, in particular a proximal end, of a rod in the surgical instrument. In particular, the rod is mounted or guided in the tubular shaft in such a way that the working channel cannot move or rotate axially relative to the tube shaft.

A tool, which can be designed in particular in the form of an interchangeable accessory, can be fluidly coupled to the accessory port.

If the surgical instrument is to be operated by a user such as a surgeon, the surgical instrument comprises a handle as a grip for the user. The operating interface then transmits a force or torque, which is applied by the user to the handle via a suitable actuation (e.g. movable handle leg), to a force transmission element for example.

If the surgical instrument is to be connected to a robot, the surgical instrument comprises a corresponding connection interface for the robot, which can apply an axial force or torque to the force transmission element via the operating interface.

Advantageous embodiments and further developments emerge from the description with reference to the figures.

According to some embodiments of the invention, the flexible shaft is configured as one piece, which is manufactured by an injection molding process. That means the flexible shaft can be manufactured in one molding process, in particular without assembling the plurality of shaft sections. Hence, manufacturing can be quicker. For example, the flexible shaft can be configured as a one-piece vertebrae produced by injection molding of plastic and comprises of single plastic material that is flexible. The plurality of shaft sections can be connected by an integrated connecting bar, which connects adjacent shaft sections such that they are articulatable in one plain.

Furthermore, a one-piece vertebrae for single use endoscope can be manufactured with injection molding. A similar design can be used for separate links vertebrae design to ease an assembly of the steering wires. The design of the flexible vertebrae can be concluded in a way that the mold would be simple and robust.

For example, each shaft section can be configured as a vertebral member, which is coupled to the adjacent vertebral member such that pivoting of the vertebral member relative to the adjacent vertebral member in a radial axis is provided.

According to some further embodiments of the invention, the plurality of shaft sections is made of a single type of plastic material, which is elastically deformable. Thus, complexity of the flexible shaft can be reduced. Furthermore, material properties and bending behavior of the flexible shaft can be determined more precisely.

For example, the flexible shaft is configured as a single use flexible endoscope one-piece vertebrae including one inner contour for all main components such as the working channel, the optical image device and the steering wires. In particular, the flexible shaft comprises the one-piece single use flexible endoscope articulation on the distal end.

According to some further embodiments of the invention, the plurality of shaft sections comprises a circular or oval shape for forming a circular or oval shaped flexible shaft. Thereby, the flexible shaft can have a constant diameter.

According to some further embodiments of the invention, the through-opening comprises a circular central area and two protruding areas, wherein the two protruding areas are each arranged on opposite sides in relation to the circular central area and are each connected to the circular central area such that a steering wire is slidable between the circular central area and the two protruding areas inside the through-opening in a radial direction of the flexible shaft.

According to some further embodiments of the invention, each of the two protruding areas is formed angled, in particular like a hook or a bayonet lock, such that the plurality of shaft sections comprises a nose element partly separating the protruding area from the circular central area. The nose element can support the steering wire when a force towards the central area is present at the steering wire. Hence, the steering wire can be kept in the protruding area during an articulation of the flexible shaft.

According to some further embodiments of the invention, the two protruding areas have a width/diameter in a range of about 0.3 mm to 0.5 mm.

According to some further embodiments of the invention, the flexible surgical instrument further comprises a working channel for communicating fluids or medical tools from the operating interface to the distal end of the flexible shaft and two steering wires extending from the operating inter-face through the through-opening for articulating the flexible shaft.

According to some further embodiments of the invention, the flexible surgical instrument further comprises an optical image device extending from the operating interface through the through-opening for generating image data of an area in front of the distal end.

According to some further embodiments of the invention, the primary shaping comprises one injection molding process, and wherein the injection molded flexible shaft is released in one piece. That means the flexible shaft can be manufactured in one molding process, in particular without assembling the plurality of shaft sections. Hence, manufacturing can be quicker.

According to some further embodiments of the invention, the second steering wire is guided through the through-opening at the same time as the first steering wire.

According to some further embodiments of the invention, the step of sliding comprises sliding the second steering wire in a radial direction of the flexible shaft for radially positioning the second steering wire opposite to the first steering wire in relation to the through-opening.

The above embodiments and further developments can be combined with one another arbitrarily, as far as appropriate. Further possible configurations, developments and implementations of the invention are also combinations of features of the invention described above or below for the exemplary embodiments that are not explicitly cited. In particular, a person skilled in the art will also add individual aspects as improvements or additions to the respective basic form of the present invention.

### BRIEF DESCRIPTION OF THE DRAWINGS

The present invention is explained more specifically below on the basis of the exemplary embodiments indicated in the schematic figures, in which:
Fig. 1 shows a schematic illustration of a flexible shaft for a flexible endoscope according to an embodiment of the invention;
Fig. 2 shows a schematic illustration of a shaft section of the flexible shaft according to Fig. 1;
Fig. 3 shows a schematic flow chart of a method for manufacturing flexible shaft for a surgical instrument according to a further embodiment of the invention;
Fig. 4 shows a schematic flow chart of a method for assembling a steering wire to a flexible shaft for a surgical instrument according to a further embodiment of the invention;
Fig. 5 shows a schematic illustration of a flexible shaft for a flexible endoscope according to another embodiment of the invention with two cross sectional views A-A and B-B;
Fig. 6 shows a schematic perspective view of a flexible shaft being configured as one piece according to another embodiment of the invention; and
Fig. 7 shows a schematic illustration of a flexible shaft for a flexible endoscope according to another embodiment of the invention.

The accompanying figures are intended to convey a further understanding of the embodiments of the invention. They illustrate embodiments and are used in conjunction with the description to explain principles and concepts of the invention. Other embodiments and many of the cited advantages emerge in light of the drawings. The elements of the drawings are not necessarily shown to scale in relation to one another. Direction-indicating terminology such as for example "at the top", "at the bottom", "on the left", "on the right", "above", "below", "horizontally", "vertically", "at the front", "at the rear" and similar statements are merely used for explanatory purposes and do not serve to restrict the generality to specific configurations as shown in the figures.

In the figures of the drawing, elements, features and components that are the same, have the same function and have the same effect are each provided with the same reference signs - unless explained otherwise.

### DETAILED DESCRIPTION OF DRAWINGS

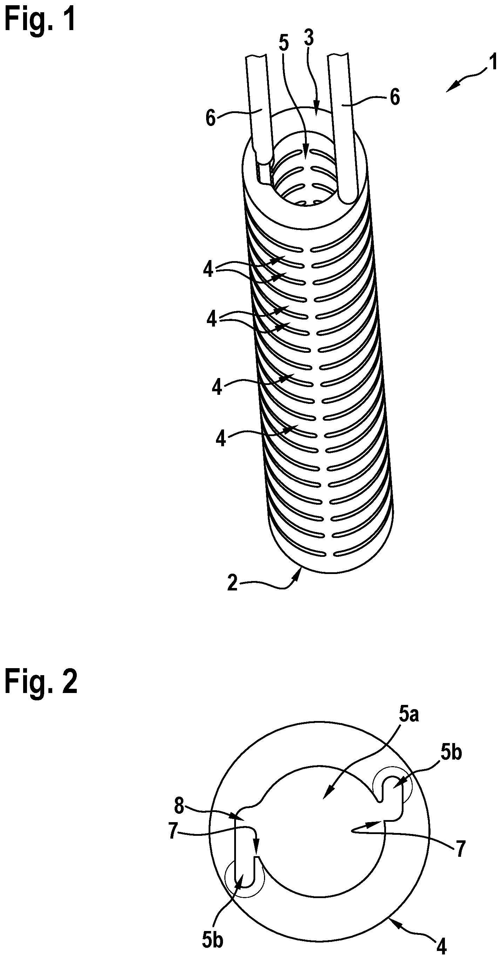

Fig. 1 shows a schematic illustration of a flexible shaft 1 for a flexible endoscope.

The flexible endoscope (not shown) can be exemplarily configured as a handheld endoscope comprising an endoscope handle for manual operation by a user. Alternatively, the endoscope handle can be configured as a corresponding interface for a robot for guidance by a robot. The flexible endoscope comprises the flexible shaft 1, which is arranged at a distal end of the flexible endoscope. The flexible endoscope may further comprise an operating interface and a shaft, which encloses a working channel, wherein the shaft is coupled to the operating interface and the flexible shaft 1. The shaft and the working channel can be configured as a round tube. The working channel is configured for communicating fluids or medical tools. Furthermore, the shaft can be arranged concentric in the operating interface.

The flexible shaft 1 comprises a distal end 2 and a proximal end 3. For example, the proximal end 3 is coupled to the shaft of the flexible endoscope. Hence, the operating interface can be arranged at the proximal end 3 of the flexible shaft 1 and configured to operate the flexible shaft 1.

Further, the flexible shaft 1 comprises a plurality of shaft sections 4, which are arranged in series between the distal end 2 and the proximal end 3. The plurality of shaft sections 4 is articulatable in one plain and comprises a through-opening 5. The plurality of shaft sections 4 is made of a single type of plastic material, which is elastically deformable. Each shaft section 4 is configured as a vertebral member, which is coupled to the adjacent vertebral member such that pivoting of the vertebral member relative to the adjacent vertebral member in a radial axis is provided. In Fig. 1, the plurality of shaft sections 4 comprises a circular shape for forming a circular shaped flexible shaft 1. Thereby, the flexible shaft 1 has a constant diameter. But the shape of the flexible shaft 1 is not limited to this and can also have an oval or similar shape.

The through-opening 5 is configured for receiving the working channel and an optical image device. In particular, the through-opening 5 is further configured for receiving two steering wires 6. Thereby, the through-opening 5 extends through the plurality of shaft sections 4 from the proximal end 3 to the distal end 2. The two steering wires 6 extend here from the operating interface of the endoscope to articulate the flexible shaft 1.

Fig. 2 shows a schematic illustration of a shaft section 4 of the flexible shaft 1 according to Fig. 1. Specifically, Fig. 2 shows a front view of the shaft section 4.

Here, the shaft section 4 comprises one through-opening 5 configured for receiving the working channel, the optical image device and the two steering wires 6. Thereby, the through-opening 5 comprises a circular central area 5a and two protruding areas 5b. The two protruding areas 5b are each arranged on opposite sides in relation to the circular central area 5a.

In Fig. 2, the two protruding areas 5b are arranged on the left side and on the right side of the shaft section 4. Additionally, the two protruding areas 5b are each connected to the circular central area 5a such that the steering wire 6 is slidable between the circular central area 5a and the two protruding areas 5b inside the through-opening 5 in a radial direction of the flexible shaft 1. One of the two protruding areas 5b, here the protruding area 5b on the left hand side, is combined with an area 8 for receiving the optical image device.

Each of the two protruding areas 5b is formed like a bayonet lock such that the plurality of shaft sections 4 or each shaft section, respectively, comprises a nose element 7 partly separating the protruding area 5b from the circular central area 5a. The nose element 7 supports the steering wire 6 when a force towards the central area 5a is present at the steering wire 6. Hence, the steering wire 6 can be kept in the protruding area 5b during an articulation of the flexible shaft 1. Here, the two protruding areas 5b have a width in a range of about 0.3 mm to 0.5 mm.

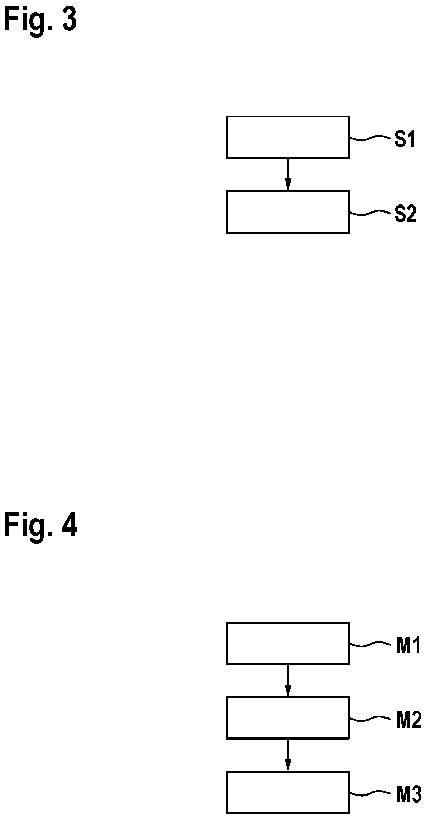

Fig. 3 shows a schematic flow chart of a method for manufacturing a flexible shaft for a surgical instrument.

The method comprises the steps of primary shaping S 1 and releasing S2.

In the step of primary shaping S 1, a plurality of shaft sections 4 is primary shaped, which are arranged in series between a distal end 2 and a proximal end 3. Furthermore, the plurality of shaft sections 4 comprise a through-opening 5, which is configured for receiving a working channel and an optical image device. The through-opening 5 is further configured for receiving at least two steering wires 6, wherein the through-opening 5 extends through the plurality of shaft sections 4 from the proximal end 3 to the distal end 2. In particular, the primary shaping S1 comprises one injection molding process.

In the step of releasing S2, the flexible shaft 1 is released from the primary shaping tool, wherein the plurality of shaft sections 4 is articulatable in one plain. Due to the one injection molding process the injection molded flexible shaft 1 is released S2 in one piece.

Fig. 4 shows a schematic flow chart of a method for assembling a steering wire 6 to a flexible shaft 1 for a surgical instrument.

The method comprises the steps of providing M1, guiding M2 and sliding M3.

In the step of providing M1, a flexible shaft 1 comprising a distal end 2, a proximal end 3, and a plurality of shaft sections 4 is provided. The plurality of shaft sections 4 are arranged in series between the distal end 2 and the proximal end 3, wherein the plurality of shaft sections 4 is articulatable at least in one plain. Further, the plurality of shaft sections 4 comprises a through-opening 5, which is configured for receiving a working channel and an optical image device. Additionally, the through-opening 5 is configured for receiving two steering wires 6, wherein the through-opening 5 extends through the plurality of shaft sections 4 from the proximal end 3 to the distal end 2.

In the step of guiding M2, a first steering wire 6 and a second steering wire 6 are guided through the through-opening 5 of the plurality of shaft sections 4 from the proximal end 3 to the distal end 2. Moreover, the second steering wire 6 is guided M2 through the through-opening 5 at the same time as the first steering wire 6.

In the step of sliding M3, the first steering wire 6 is slid in a radial direction of the flexible shaft 1 for radially positioning the first steering wire 6 in relation to the through-opening 5. Furthermore, the second steering wire 6 is slid in a radial direction of the flexible shaft 1 for radially positioning the second steering wire 6 opposite to the first steering wire 6 in relation to the through-opening 5.

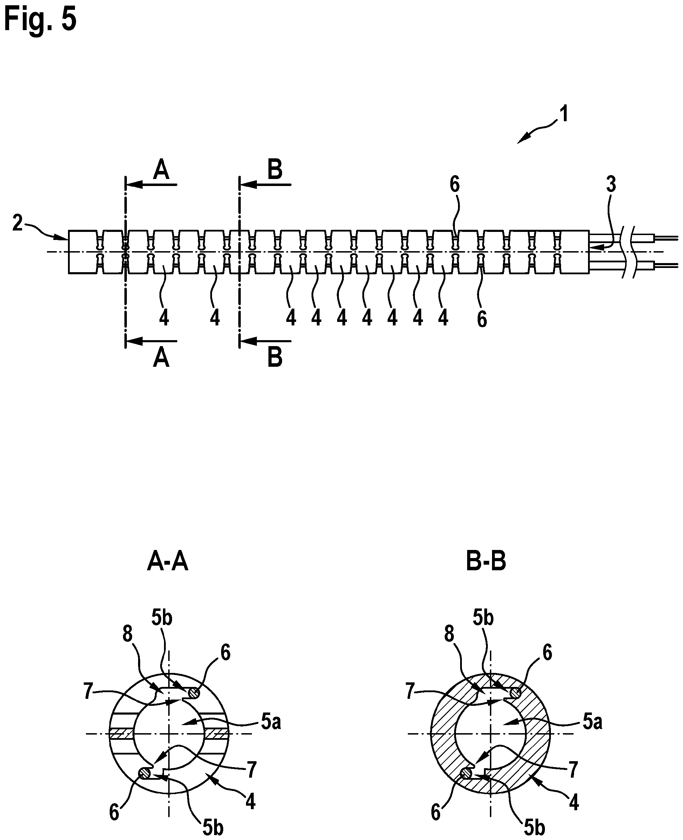

Fig. 5 shows a schematic illustration of a flexible shaft 1 for a flexible endoscope with two cross sectional views A-A and B-B.

The flexible shaft 1 comprises a distal end 2 and a proximal end 3. For example, the proximal end 3 is coupled to the shaft of the flexible endoscope. Hence, the operating interface can be arranged at the proximal end 3 of the flexible shaft 1 and configured to operate the flexible shaft 1.

Further, the flexible shaft 1 comprises a plurality of shaft sections 4, which are arranged in series between the distal end 2 and the proximal end 3. The plurality of shaft sections 4 is articulatable in one plain and comprises a through-opening 5. The plurality of shaft sections 4 is made of a single type of plastic material, which is elastically deformable. Each shaft section 4 is configured as a vertebral member, which is coupled to the adjacent vertebral member such that pivoting of the vertebral member relative to the adjacent vertebral member in a radial axis is provided.

In detail, the cross sectional view A-A illustrates the cross section of the flexible shaft between two adjacent shaft sections 4. The cross sectional view B-B illustrates the cross section of the shaft section 4.

In view A-A it is clearly shown that adjacent shaft sections 4 are linked together such that they can swivel relative to each other in one plain. When each linkage between adjacent shaft sections 4 is in the same radial orientation like it is illustrated in Fig. 5 the flexible shaft 1 is articulatable in one plain. When at least one linkage between adjacent shaft sections 4 has a different radial orientation compared to some other linkages the flexible shaft 1 is articulatable in at least two plains.

Moreover, two steering wires 6 are received in the through-opening 5. The through-opening 5 and the two steering wires 6 extend through the plurality of shaft sections 4 from the proximal end 3 to the distal end 2. The through-opening 5 is further configured for receiving a working channel and an optical image device.

Thereby, the through-opening 5 comprises a circular central area 5a and two protruding areas 5b. The two protruding areas 5b are each arranged on opposite sides in relation to the circular central area 5a.

In the two cross sectional views A-A and B-B, the two protruding areas 5b are arranged on the upper side and on the lower side of the shaft section 4. Additionally, the two protruding areas 5b are each connected to the circular central area 5a such that the steering wire 6 is slidable between the circular central area 5a and the two protruding areas 5b inside the through-opening 5 in a radial direction of the flexible shaft 1. One of the two protruding areas 5b, here the protruding area 5b on the upper hand side, is combined with an area 8 for receiving the optical image device.

Each of the two protruding areas 5b is formed like a bayonet lock such that the plurality of shaft sections 4 or each shaft section, respectively, comprises a nose element 7 partly separating the protruding area 5b from the circular central area 5a. The nose element 7 supports the steering wire 6 when a force towards the central area 5a is present at the steering wire 6. Hence, the steering wire 6 can be kept in the protruding area 5b during an articulation of the flexible shaft 1. Here, the two protruding areas 5b have a width in a range of about 0.3 mm to 0.5 mm.

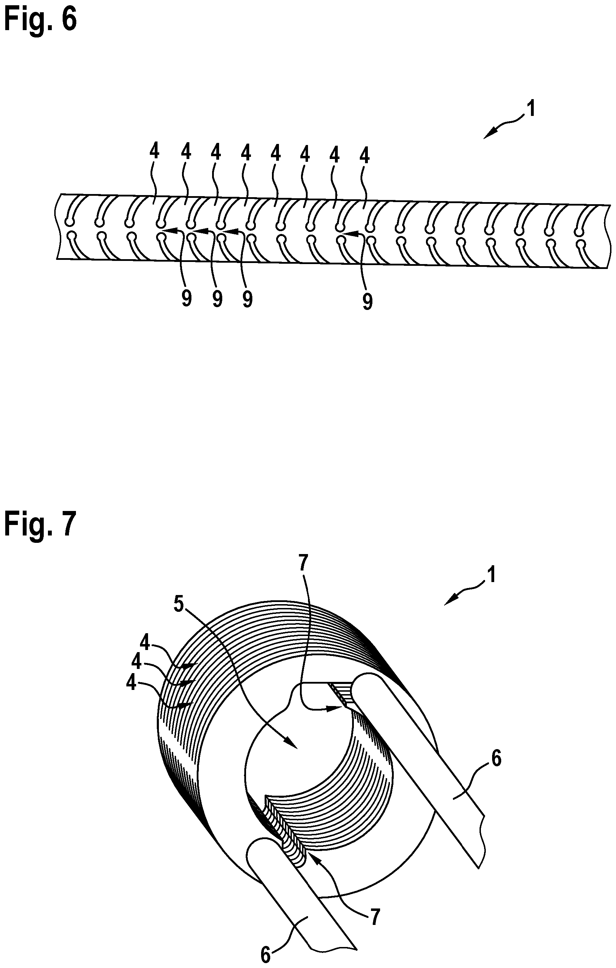

Fig. 6 shows a schematic perspective view of a flexible shaft 1 being configured as one piece. The flexible shaft of Fig. 6 substantially comprises the same features as the flexible shaft 1 of Fig. 1 or 5.

Here, the flexible shaft 1 differs from Fig. 1 or 5 in that it is configured as one piece, which is manufactured by an injection molding process. That means the flexible shaft 1 can be manufactured in one molding process without assembling the plurality of shaft sections 4.

Thereby, the plurality of shaft sections 4 is connected by an integrated connecting bar 9, which connects adjacent shaft sections 4 such that they are articulatable in one plain.

Fig. 7 shows a schematic illustration of a flexible shaft 1 for a flexible endoscope.

The flexible shaft of Fig. 7 substantially comprises the same features as the flexible shaft 1 of Fig. 1 or 5. In Fig. 7, the flexible shaft 1 and the through-opening 5, in particular the central area 5a, have a constant diameter. But the shape of the flexible shaft 1 is not limited to this and can also have an oval or similar shape. Also, the shape of the through-opening 5, in particular the central area 5a, is not limited to this and can also have an oval or similar shape. For example, the flexible shaft 1 and the through-opening 5 can have the same shape with a different diameter or width. Alternatively, the flexible shaft 1 and the through-opening 5, in particular the central area 5a, can have different shapes.

In the detailed description above, various features have been combined in one or more examples in order to improve the rigorousness of the illustration. However, it should be clear in this case that the above description is of merely illustrative but in no way restrictive nature. It serves to cover all alternatives, modifications and equivalents of the various features and exemplary embodiments. Many other examples will be immediately and directly clear to a person skilled in the art on the basis of his knowledge in the art in consideration of the above description.

The exemplary embodiments have been chosen and described in order to be able to present the principles underlying the invention and their application possibilities in practice in the best possible way. As a result, those skilled in the art can optimally modify and utilize the invention and its various exemplary embodiments with regard to the intended purpose of use. In the claims and the description, the terms "including" and "having" are used as neutral linguistic concepts for the corresponding terms "comprising". Furthermore, use of the terms "a", "an" and "one" shall not in principle exclude the plurality of features and components described in this way.

While at least one exemplary embodiment of the present invention(s) is disclosed herein, it should be understood that modifications, substitutions and alternatives may be apparent to one of ordinary skill in the art and can be made without departing from the scope of this disclosure. This disclosure is intended to cover any adaptations or variations of the exemplary embodiment(s). In addition, in this disclosure, the terms "comprise" or "comprising" do not exclude other elements or steps, the terms "a" or "one" do not exclude a plural number, and the term "or" means either or both. Furthermore, characteristics or steps which have been described may also be used in combination with other characteristics or steps and in any order unless the disclosure or context suggests otherwise. This disclosure hereby incorporates by reference the complete disclosure of any patent or application from which it claims benefit or priority.

### REFERENCE LIST

- 1: flexible shaft
- 2: distal end
- 3: proximal end
- 4: plurality of shaft sections
- 5: through-opening
- 5a: circular central area
- 5b: protruding area
- 6: steering wire
- 7: nose element
- 8: area
- 9: connecting bar

- M1: providing
- M2: guiding
- M3: sliding
- S 1: primary shaping
- S2: releasing

## Claims

1. Flexible shaft (1) for a surgical instrument, in particular for a flexible endoscope, comprising:
a distal end (2) and a proximal end (3); and
a plurality of shaft sections (4), which are arranged in series between the distal end (2) and the proximal end (3);
wherein the plurality of shaft sections (4) is articulatable at least in one plain and comprises a through-opening (5), which is configured for receiving a working channel and an optical image device, wherein the through-opening (5) is further configured for receiving at least two steering wires (6), wherein the through-opening (5) extends through the plurality of shaft sections (4) from the proximal end (3) to the distal end (2).

2. Flexible shaft (1) according to claim 1,
**characterized in that**
the flexible shaft (1) is configured as one piece, which is manufactured by an injection molding process.

3. Flexible shaft (1) according to claim 1 or 2,
**characterized in that**
the plurality of shaft sections (4) is made of a single type of plastic material, which is elastically deformable.

4. Flexible shaft (1) according to one of the preceding claims,
**characterized in that**
the plurality of shaft sections (4) comprises a circular or oval shape for forming a circular or oval shaped flexible shaft.

5. Flexible shaft (1) according to one of the preceding claims,
**characterized in that**
the through-opening (5) comprises a circular central area (5a) and two protruding areas (5b), wherein the two protruding areas (5b) are each arranged on opposite sides in relation to the circular central area (5a) and are each connected to the circular central area (5a) such that a steering wire (6) is slidable between the circular central area (5a) and the two protruding areas (5b) inside the through-opening (5) in a radial direction of the flexible shaft (1).

6. Flexible shaft (1) according to claim 5,
**characterized in that**
each of the two protruding areas (5b) is formed angled, in particular like a hook or a bayonet lock, such that the plurality of shaft sections (4) comprises a nose element (7) partly separating the protruding area (5b) from the circular central area (5a).

7. Flexible shaft (1) according to claim 5 or 6,
**characterized in that**
the two protruding areas (5b) have a width/diameter in a range of about 0.3 mm to 0.5 mm.

8. Flexible surgical instrument, in particular flexible endoscope, comprising:
a flexible shaft (1) according to one of the preceding claims arranged at a distal end of the flexible surgical instrument;
an operating interface, which is arranged at the proximal end (3) of the flexible shaft (1) and configured to operate the flexible shaft (1).

9. Flexible surgical instrument according to claim 8,
**characterized in that**
the flexible surgical instrument further comprises a working channel for communicating fluids or medical tools from the operating interface to the distal end (2) of the flexible shaft (1) and two steering wires (6) extending from the operating interface through the through-opening (5) for articulating the flexible shaft (1).

10. Flexible surgical instrument according to claim 8 or 9,
**characterized in that**
the flexible surgical instrument further comprises an optical image device extending from the operating interface through the through-opening (5) for generating image data of an area in front of the distal end (2).

11. Method for manufacturing a flexible shaft for a surgical instrument, in particular for manufacturing a flexible shaft (1) according to one of the claims 1 to 7, comprising the steps of:
primary shaping (S1) of a plurality of shaft sections (4), which are arranged in series between a distal end (2) and a proximal end (3) and comprise a through-opening (5), which is configured for receiving a working channel and an optical image device, wherein the through-opening (5) is further configured for receiving at least two steering wires (6), wherein the through-opening (5) extends through the plurality of shaft sections (4) from the proximal end (3) to the distal end (2); and
releasing (S2) the flexible shaft (1) from the primary shaping tool, wherein the plurality of shaft sections (4) is articulatable at least in one plain.

12. Method according to claim 11,
**characterized in that**
the primary shaping (S1) comprises one injection molding process, and wherein the injection molded flexible shaft is released (S2) in one piece.

13. Method for assembling a steering wire to a flexible shaft for a surgical instrument, in particular to a flexible shaft (1) according to one of the claims 1 to 7, comprising the steps of:
providing (M1) a flexible shaft (1) comprising a distal end (2), a proximal end (3), and a plurality of shaft sections (4), which are arranged in series between the distal end (2) and the proximal end (3), wherein the plurality of shaft sections (4) is articulatable at least in one plain and comprises a through-opening (5), which is configured for receiving a working channel and an optical image device, wherein the through-opening (5) is further configured for receiving at least two steering wires (6), wherein the through-opening (5) extends through the plurality of shaft sections (4) from the proximal end (3) to the distal end (2);
guiding (M2) a first steering wire (6) and a second steering wire (6) through the through-opening (5) of the plurality of shaft sections (4) from the proximal end (3) to the distal end (2); and
sliding (M3) the first steering wire (6) in a radial direction of the flexible shaft (1) for radially positioning the first steering wire in relation to the through-opening (5).

14. Method according to claim 13,
**characterized in that**
the second steering wire (6) is guided (M2) through the through-opening (5) at the same time as the first steering wire (6).

15. Method according to claim 13 or 14,
**characterized in that**
the step of sliding (M3) comprises sliding the second steering wire (6) in a radial direction of the flexible shaft (1) for radially positioning the second steering wire (6) opposite to the first steering wire (6) in relation to the through-opening (5).
